# EUROPÄISCHE PATENTSCHRIFT

(11) **EP 0 857 713 B1**
(45) Veröffentlichungstag und Bekanntmachung des Hinweises auf die Patenterteilung: **13.06.2001**
(21) Anmeldenummer: 98101693.4
(22) Anmeldetag: 02.02.1998
(51) Int. Cl.: C07C 65/03, C07C 69/157, C07C 43/225, C07C 65/24, C07F 3/02

(54) **Verfahren zur Herstellung von 3-Hydroxy-2-methylbenzoesäure und 3-Acetoxy-2-methylbenzoesäure**
Process for the preparation of 3-hydroxy- and 3-acetoxy-2-methylbenzoic acid
Procédé pour la préparation de l'acide 3-hydroxy- et 3-acétoxy-2-méthylbenzoique

(30) Priorität: 11.02.1997 DE 19704885
(43) Veröffentlichungstag der Anmeldung: 12.08.1998
(73) Patentinhaber: Clariant GmbH, 65929 Frankfurt am Main (DE)
(72) Erfinder: Cosmo, Robert, Dr., 64291 Darmstadt (DE); Dierdorf, Andreas, Dr., 60529 Frankfurt (DE)

(56) Entgegenhaltungen:
- WO-A-95/21164
- US-A- 5 484 926
- T. M. CRESP: "Synthesis of piloquinone, a metabolite of Streptomyces Pilosus Ettlinger" JOURNAL OF THE CHEMICAL SOCIETY, PERKIN TRANSACTIONS 1, 1974, LETCHWORTH GB, Seiten 2435-2447, XP002064503

## Beschreibung

Die Erfindung betrifft ein Verfahren zur Herstellung von 3-Hydroxy-2-methylbenzoesäure und 3-Acetoxy-2-methylbenzoesäure.

3-Acetoxy-2-methylbenzoesäure (1) ist ein Vorprodukt für HIV Protease Inhibitoren, die im US Patent 5.484.926 beschrieben sind.

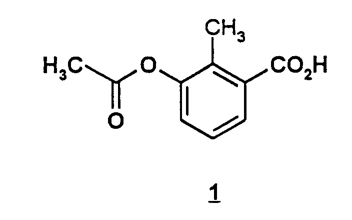

3-Acetoxy-2-methylbenzoesäure wird aus 3-Hydroxy-2-methylbenzoesäure (2) mit überschüssigem Acetanhydrid im Gegenwart einer Mineralsäure hergestellt. (US Patent 5.484.926, Spalte 111, Beispiel 81).

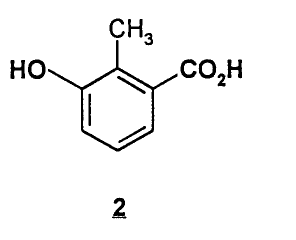

Die Verbindung 3-Hydroxy-2-methylbenzoesäure selbst ist ein Schlüsselverbindung für eine Reihe von anderen HIV Protease Inhibitoren. Einige von diesen Wirkstoffen sind in WO 95/21164, WO 95/32185 und WO 96/22287 beschrieben.

Über zahlreiche Synthesen von 3-Hydroxy-2-methylbenzoesäure wurden bisher berichtet. Alle bisherigen Synthesen von 3-Hydroxy-2-methylbenzoesäure haben jedoch erhebliche wirtschaftliche und technologische Nachteile.

Nach DRP 91.201 wird 3-Hydroxy-2-methylbenzoesäure durch das Verschmelzen von 1,3,5-Naphthalintrisulfonsäure mit einem sehr großen Überschuß 50%iger Natronlauge in einem Autoklaven bei 260°C und anschließender saurer Aufarbeitung hergestellt. Bei dieser Reaktion können auch Naphthalinderivate, in denen eine oder zwei der Sulfogruppen von 1,3,5-Naphthalintrisulfonsäure durch OH bzw. NH₂ ersetzt sind, eingesetzt werden. Besonders nachteilig an diesen Verfahren sind die sehr geringen Ausbeuten: Zum Beispiel wird aus dem Dinatrium-Salz der 2-Naphthylamin-4,8-disulfonsäure nur 18-27% Ausbeute an 3-Hydroxy-2-methylbenzoesäure isoliert [Fieser et al. J.Am.Chem.Soc., 58, 749 (1936)]. Zusätzliche Nachteile an diesem Verfahren sind die drastischen Reaktionsbedindungen und der hohe Anfall an unweltbelastendem salzhaltigem Abwasser.

Ein weiteres Verfahren geht von 3-Chlor-2-methylphenol aus (Cresp et al., J.Chem.Soc.Perkin Trans.1, 2435(1974). Dies wird mit Kupfer(I)cyanid in Pyridin in der Siedehitze zu 3-Hydroxy-2-methylbenzonitril umgesetzt. In der zweiten Stufe wird das Nitril in einem siedenden Gemisch aus Wasser, Eisessig und konzentrierter Schwefelsäure in 18 Stunden zu 3-Hydroxy-2-methylbenzoesäure (2) verseift. Die erste Stufe dieses Verfahrens hat den wesentlichen Nachteil, daß das sehr giftige Kupfer(I)cyanid eingesetzt wird - unter den Reaktionsbedingungen kann giftiges Cyanwasserstoff-Gas aus dem Reaktionsgemisch entweichen. Als Lösungsmittel wird das gesundheitsschädliche Pyridin eingesetzt. Die Reaktion zeichnet sich durch eine schwierige Aufarbeitung und einen hohen Anfall an giftigen Salzen aus. Bei der zweiten Stufe (Amidverseifung) ist eine sehr lange Reaktionszeit erforderlich. Bei den sehr hohen Reaktionstemperaturen ist das saure Reaktionsmedium sehr korrosiv gegenüber den üblicherweise in Chemieanlagen eingesetzten Werkstoffen.

In Moreau et al., Bull.Soc.chim.Fr., 3427 (1973) ist die Herstellung von 3-Hydroxy-2-methyl-benzoesäure ausgehend von 2-Methyl-3-nitrobenzoesäure beschrieben. 2-Methyl-3-nitro-benzoesäure wird durch katalytische Reduktion über Palladium-Kohle zu 3-Amino-2-methylbenzoesäure reduziert. Nach Diazotierung dieses Aminosäure-Derivats und Verkochung des Diazonium-Salzes wird 3-Hydroxy-2-methylbenzoesäure erhalten.

Der Nachteil dieses Syntheseweges liegt in der schlechten Verfügbarkeit des Ausgangsstoffes 2-Methyl-3-nitrobenzoesäure. In Kulic et al., J.Gen.Chem. USSR (Engl.), 60, 2118 (1990) wird die Oxidation von 2,3-Dimethylnitrobenzol zu 2-Methyl-3-nitrobenzoesäure mit wäßrigem Kaliumpermanganat im Gegenwart eines Phasentransferkatalysators bei 75°C durchgeführt. Bei diesem Verfahren fällt 2-Methyl-3-nitrobenzoesäure nicht als Reinprodukt an, sondern als Gemisch mit 3-Nitrophthalsäure und geringeren Mengen an 3-Methyl-2-nitrobenzoesäure. Eine aufwendige Reinigung ist notwendig, um reine 2-Methyl-3-nitrobenzoesäure aus dem Rohprodukt zu erhalten. Dies geht auf Kosten der Ausbeute. Ein weiterer gravierender Nachteil ist die extrem schlechte Raumausbeute der Oxidationsreaktion. Pro 100 ml Wasser werden nur 2.4 g 2,3-Dimethylnitrobenzol eingesetzt (siehe S. 2121). Zudem fällt Braunstein bei der Permanganat-Oxidation an, das entsorgt werden muß.

Auch die Oxidation von 2,3-Dimethylnitrobenzol mit anderen Oxidationsmitteln verläuft unbefriedigend. Z.B. liefert die Oxidation von 2,3-Dimethylnitrobenzol mit wäßriger Salpetersäure die gewünschte 2-Methyl-3-nitrobenzoesäure in nur 46.6% Ausbeute (US Patent 4.065.477, Spalte 4).

Eine alternative Herstellung von 2-Methyl-3-nitrobenzoesäure beinhaltet die Nitrierung von o-Tolylsäure (Giacolone, Gazz.Chem.Ital., 65, 840 (1935)). Bei dieser Nitrierung fällt jedoch die isomere 2-Methyl-5-nitrobenzoesäure als Hauptprodukt an. Pro Teil 2-Methyl-3-nitrobenzoesäure werden 2 Teile 2-Methyl-5-nitrobenzoesäure gebildet. Auf Grund des schlechten Selektivät der Nitrierung zu 2-Methyl-3-nitrobenzoesäure ist diese Synthese nicht geeignet, um 2-Methyl-3-nitrobenzoesäure kostengünstig verfügbar zu machen.

Eine weitere Synthese von 3-Hydroxy-2-methylbenzoesäure (2) geht von 3-Methoxybenzoylchlorid aus (WO-A-9 521 164). 3-Methoxybenzoylchlorid wird mit Anilin zu 3-Methoxy-N-phenylbenzamid umgesetzt. Umsetzung von 3-Methoxy-N-phenylbenzamid mit 2 Äquivalenten n-Butyllithium und anschließende Alkylierung mit Methyljodid liefert 3-Methoxy-2-methyl-N-phenylbenzamid. Die Reaktion von 3-Methoxy-2-methyl-N-phenylbenzamid mit wäßriger Salzsäure und wäßriger Bromwasserstoffsäure in siedender Essigsäure führt zur Verseifung der Amidfunktion und zur Spaltung der Methoxygruppe und zur Bildung von 3-Hydroxy-2-methylbenzoesäure. Ein Nachteil der Reaktionssequenz besteht darin, daß sehr tiefe Temperaturen (-15° bis -70°) bei der zweiten Stufe benötigt werden. Solche Temperaturen sind bei der Durchführung der Reaktion in technischem Maßstab schwer realisierbar. Bei der dritten Stufen sind sehr aggressive Reaktionsbedingungen notwendig, um die Spaltung der Methoxy-Gruppe zu bewerkstelligen. Zudem entstehen die gasförmigen Stoffe Methylchlorid bzw. Methylbromid als zwangsläufige Nebenprodukte der Spaltung, die in Verdacht stehen, krebserzeugend zu sein. Außerdem wirkt das saure Reaktionsmedium sehr korrosiv gegenüber den üblicherweise in Chemieanlagen eingesetzten Werkstoffen.

Es bestand somit ein großer Bedarf nach einem Verfahren, das es erlaubt, 3-Hydroxy-2-methylbenzoesäure und 3-Acetoxy-2-methylbenzoesäure auf wirtschaftliche und technisch einfache Weise zu erhalten.

Diese Aufgabe wird gelöst durch ein Verfahren zur Herstellung von 3-Hydroxy-2-methylbenzoesäure und 3-Acetoxy-2-methylbenzoesäure, dadurch gekennzeichnet, daß man 3-Chlor-2-methylphenol (3) 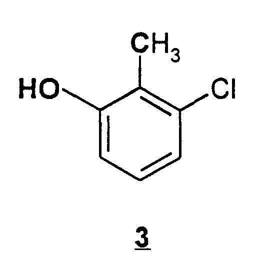 mit Benzylchlorid zu 2-Benzyloxy-6-chlortoluol (4) 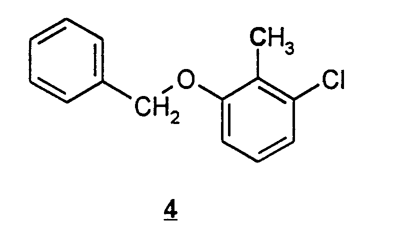 umsetzt, dieses mit Magnesium zu (3-Benzyloxy-2-methylphenyl)-magnesiumchlorid (5) 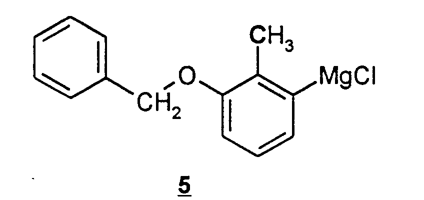 grignardisiert, dieses mit CO₂ zu 3-Benzyloxy-2-methylbenzoesäure (6) 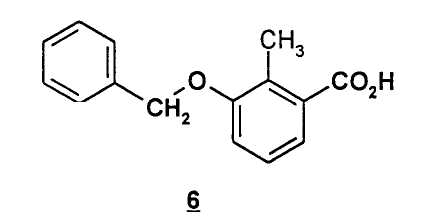 umsetzt, dieses oder seine Alkalimetallsalze im Gegenwart eines Hydrierungskatalysators zu 3-Hydroxy-2-methylbenzoesäure (2) hydriert und dieses gegebenenfalls zu 3-Acetoxy-2-methylbenzoesäure acetyliert.

Die Erfindung betrifft ebenfalls die Zwischenprodukte 2-Benzyloxy-6-chlortoluol (4), (3-Benzyloxy-2-methylphenyl)-magnesiumchlorid (5) und 3-Benzyloxy-2-methylbenzoesäure (6).

Das Verfahren hat die wesentlichen Vorteile, daß es kostengünstig ist und daß der Anfall an Abfallprodukten vergleichsweise gering ist. 3-Chlor-2-methylphenol kann aus dem billigen Ausgangsstoff 3-Chlor-2-methylanilin durch Diazotierung und anschließende Verkochung in hoher Ausbeute dargestellt werden. Ein solches Verfahren ist in Noelting et al., Chem. Berichte, 37, 1015, (1904) beschrieben.

Die Alkylierung von 3-Chlor-2-methylphenol zu 2-Benzyloxy-6-chlortoluol kann in einer Reihe von Lösungsmitteln, z.B. Aceton, Methylethylketon, Dimethylformamid durchgeführt werden. Besonders bewährt hat sich die Verwendung von Methylethylketon. Pro Mol 3-Chlor-2-methylphenol setzt man zweckmäßigerweise 1.0 bis 5.0 Mol, bevorzugt 1.1 bis 1.2 Mol Benzylchlorid ein. Die Alkylierungsreaktion wird vorteilhaft in Gegenwart von mindestens 1 Mol Base wie Natriumhydroxid oder Kaliumcarbonat pro Mol 3-Chlor-2-methylphenol durchgeführt. Günstig ist die Verwendung von Kaliumcarbonat. Pro Mol 3-Chlor-2-methylphenol setzt man vorteilhaft 1.0 bis 5.0 Mol, bevorzugt 2.0 bis 2.5 Mol Kaliumcarbonat ein. Die produkthaltige organische Lösung wird von Kaliumcarbonat filtriert und destillativ aufgearbeitet.

Die Grignardisierung von 2-Benzyloxy-6-chlortoluol mit Magnesium zu (3-Benzyloxy-2-methylphenyl)-magnesiumchlorid wird nach üblichen Methoden durchgeführt (siehe Ullmann's Encyclopedia of Industrial Chemistry, 5. Auflage, Band A15, Seiten 625-626). Es hat sich bewährt, die Grignardisierung in Tetrahydrofuran (THF) als Reaktionsmedium durchzuführen.

Die Umsetzung von (3-Benzyloxy-2-methylphenyl)-magnesiumchlorid mit Kohlendioxid zu 3-Benzyloxy-2-methylbenzoesäure kann so durchgeführt werden, daß überschüssiges Kohlendioxid-Gas in die Lösung des Grignard-Reagenzes (5) in THF eingeleitet wird. Es besteht die Alternative, THF vorzulegen, während Kohlendioxid-Gas eingeleitet und die Grignard-Lösung zudosiert wird. Dabei wird zunächst eine Suspension des Magnesiumchlorid-Salzes der Säure (6) gebildet. Das Salz wird mit ausreichender wäßriger Mineralsäure, bevorzugt wäßriger Salzsäure hydrolysiert, so daß die freie Säure (6) vollständig freigesetzt wird.

Nach der Hydolyse wird die produkthaltige THF-Phase von der salzhaltigen wäßrigen Phase abgetrennt. Die Isolierung der Säure aus der Lösung kann auf verschiedene Weisen erfolgen.

Bei der Carboxylierung vom Grignard-Reagenz (5) sind verschiedene weitere Verfahrensvarianten möglich. Das Kohlendioxid kann auch in fester Form eingesetzt werden. Dabei ist es möglich, Trockeneis portionsweise in die Grignard-Lösung einzutragen bzw. das Trockeneis in dem Reaktionsmedium vorzulegen und die Lösung des Grignard-Reagenz zuzudosieren. Kohlendioxid kann auch in flüssiger Form eingesetzt werden (siehe z.B. BP 1.028.599).
Die Hydrogenolyse von 3-Benzyloxy-2-methylbenzoesäure zu 3-Hydroxy-2-methylbenzoesäure und Toluol wird bevorzugt in wäßrigem Medium durchgeführt. Um die Löslichkeit von 3-Benzyloxy-2-methylbenzoesäure in Wasser zu verbessern, ist es dabei vorteilhaft, mindestens 1.0 Mol einer starken Base wie Natriumhydroxid oder Kaliumhydroxid (bevorzugt Natriumhydroxid) pro Mol 3-Benzyloxy-2-methylbenzoesäure zum Reaktionsgemisch zuzusetzten, so daß das besser wasserlösliche Alkalimetallsalz der Carbonsäure gebildet wird. Die Base kann entweder als Feststoff oder als wäßrige Lösung zugesetzt werden. Die Reaktion kann allerdings auch in organischen Medien unter neutralen Bedingungen durchgeführt werden. Die Hydrogenolyse verläuft bereits bei Raumtemperatur und bei mäßigem Wasserstoff-Druck (1 - 10 bar). Bei der Fahrweise im wäßrigen basischen Medium wird der Katalysator nach beendeter Hydrogenolyse abfiltriert. Die produkthaltige wäßrige Phase wird von der Toluol-Phase getrennt und angesäuert. Die ausgefallene 3-Hydroxy-2-methylbenzoesäure wird durch Filtration isoliert.

Die üblichen Hydrierungskatalysatoren wie z.B. Palladium auf Aktivkohle, Platin auf Aktivkohle oder Raney-Nickel können bei der Hydrogenolyse eingesetzt werden. Bewährt hat sich die Verwendung von Palladium auf Aktivkohle.
Die Durchführung der Hydrogenolyse in wäßrigem Medium eröffnet die vorteilhafte Möglichkeit, die katalytische Reduktion von 3-Benzyloxy-2-methylbenzoesäure zu 3-Hydroxy-2-methyl-benzoesäure und die Acetylierung zu 3-Acetoxy-2-methylbenzoesäure ohne Zwischenisolierung der Hydroxysäure (2) durchzuführen.

Die Hydrogenolyse wird in diesem Fall zweckmäßigerweise in Gegenwart von mindestens 1.0 Mol der starken Base (bevorzugt 2.0 bis 3.0 Mol) pro Mol 3-Benzyloxy-2-methylbenzoesäure durchgeführt. Bevorzugt wird Natriumhydroxid als Base eingesetzt. Nach Abfiltrieren des Katalysators und Abtrennung der organischen Toluol-Phase wird die produkthaltige wäßrige Phase mit Acetanhydrid behandelt um die Acetylierung zu bewerkstelligen. Dabei ist es vorteilhaft, 1.0 bis 3.0 Mol Acetanhydrid, bevorzugt 1.2 bis 1.6 Mol, pro Mol eingesetzte 3-Benzyloxy-2-methylbenzoesäure einzusetzen. Die wäßrige Fahrweise ermöglicht eine sehr einfach Aufarbeitung und Produktisolierung: Nach dem Ansäuren des Reaktionsgemisches mit einer starken Mineralsäure wie Salzsäure, fällt 3-Acetoxy-2-methylbenzoesäure als Niederschlag aus Wasser aus, der durch Filtration in hoher Ausbeute isoliert wird. Es ist aber auch möglich, die Toluol-Phase erst nach der Umsetzung mit Acetanhydrid abzutrennen.

### Beispiele:

### 2-Benzyloxy-6-chlortoluol

Unter Rühren wird ein Gemisch aus 143 g (1.0 Mol) 3-Chlor-2-methylphenol, 139 g (1.1 Mol) Benzylchlorid, 276 g (2.0 Mol) Kaliumcarbonat, und 680 g Methylethylketon (MEK) unter Rückfluß erhitzt. Nach 8 Stunden werden weitere 25 g (0.18 Mol) Kaliumcarbonat zugegeben und dann weitere 8 Stunden unter Rückfluß erhitzt. Der Ansatz wird filtriert und der Filterkuchen mit MEK gewaschen. Nach destillativer Aufarbeitung werden 206 g 2-Benzyloxy-6-chlortoluol (Kp. 161°C, 3 mbar) alsblaßgelbe Flüssigkeit in 99.6% Reinheit (Flächen-% nach GC) erhalten. Dies entspricht einer Ausbeute von 88.3 % d.Th.
¹H-Nmr (CDCl₃, 60 MHz): δ (ppm): 6.6-7.4 (m, 8H); 5.0 (s, 2H); 2.3 (s, 3H).
GC-MS: M⁺ = 232

### 3-Benzyloxy-2-methylbenzoesäure

232,7 g 2-Benzyloxy-6-chlortoluol (232,7 g, 1.0 Mol) wurden mit 26.7 g (1.1 Mol) Magnesium-Späne in 550 ml THF grignardisiert. Die daraus gebildete braunschwarze Lösung von (3-Benzyloxy-2-methylphenyl)-magnesiumchlorid in THF wird durch ein Eisbad auf 0°C kaltgerührt. CO₂-Gas wird so langsam in das gerührte Reaktionsgemisch eingeleitet, sodaß die Temperatur 10°C nicht übersteigt. Die daraus gebildete Feststoffsuspension in THF wird unter Kühlung durch langsame Zugabe von 50 ml Wasser und 445 g (1.2 Mol) Salzsäure (10%-ig) hydrolysiert. Das hydrolysierte Reaktionsgemisch wird mit 430 g Xylol verdünnt.

Die Phasen werden getrennt und die organische Phase wird mit 100 g Wasser gewaschen.
Die abgetrennte produkthaltige organische Phase wird destilliert bis der Sumpf frei von THF ist. Der Sumpf wird anschließend auf 0°C kaltgerührt. Das auskristallisierte Produkt wird abfiltriert und mit 50 ml Xylol gewaschen. Nach Trocknung im Vakuum werden 152.3 g 3-Benzyloxy-2-methylbenzoesäure erhalten. Dies entspricht einer Ausbeute von 62,9 % d.Th.
Schmelzpunkt: 126-127°C
¹H-Nmr (DMSO-d₆, 60 MHz): δ (ppm): 12.9 (bs, 1H); 7.6-7.1 (m, 8H); 5.1 (s, 2H); 2.4 (s, 3H).
GC-MS: M⁺ = 242

### 3-Hydroxy-2-methylbenzoesäure

Eine Lösung von 3-(Benzyloxy)-2-methylbenzoesäure (50 g, 0,21 mol) und Natriumhydroxid (9 g, 0,225 mol) in 700 g Wasser sowie 2 g 5% Pd/C-Katalysator (50% wasserfeucht) werden in einen 2L-Stahlautoklaven mit Begasungsrührer gegeben. Bei einer Temperatur von 50°C und einer Rührgeschwindigkeit von 800 U/min werden 10 bar Wasserstoff aufgepreßt. Verbrauchter Wasserstoff wird durch wiederholtes Aufpressen ersetzt. Nach 20 min läßt die Wasserstoffaufnahme nach. Der Katalysator wird bei Raumtemperatur über eine Drucknutsche abfiltriert. Man erhält 760,7 g einer Rohlösung, von der im Scheidetrichter 7,7 g Toluol abgetrennt werden. Zu der Rohlösung werden insgesamt 22,2 g Salzsäure (37%ig) gegeben, anschließend wird die Lösung auf 0°C abgekühlt. Der ausgefallene Feststoff wird abgesaugt und zweimal mit 50 g Wasser gewaschen. Nach dem Trocknen erhält man 18,0 g 3-Hydroxy-2-methylbenzoesäure mit einem Schmelzpunkt von 147°C. Nach Einengen der Mutterlauge können nochmals 9,6 g Substanz gewonnen werden, so daß die Gesamtausbeute 27,6 g oder 88,1 % der Theorie beträgt.

### 3-Acetoxy-2-methylbenzoesäure

Unter Rühren werden 60.9 g (0.40 mol) 3-Hydroxy-2-methylbenzoesäure in 500 ml 2N Natronlauge-(1.00 Mol) eingetragen und die dabei gebildete Lösung wird auf 5°C gekühlt. In die gekühlte Lösung werden 61.3 g (0.60 Mol) Acetanhydrid zudosiert. Dabei kühlt man das Reaktionsgemisch, so daß die Temperatur 8°C nicht übersteigt. Das Reaktionsgemisch wird dann mit 88 ml 37% Salzsäure angesäuert. Das ausgefallene Produkt wird abfiltriert, dreimal mit 50 ml Wasser gewaschen und im Vakuum getrocknet. Es werden 69.9 g 3-Acetoxy-2-methylbenzoesäure erhalten. Dies entspricht einer Ausbeute von 90,0 % d.Th.
Schmelzpunkt 147°-148°C

### 3-Acetoxy-2-methylbenzoesäure durch Hydrogenolyse von 3-Benzyloxy-2-methylbenzoesäure und anschließende Acetylierung ohne Zwischenisolierung von 3-Hydroxy-2-methylbenzoesäure

Eine Lösung von 3-Benzyloxy-2-methylbenzoesäure (50 g, 0,21 mol) und Natriumhydroxid (20 g, 0,5 mol) in 700 g Wasser sowie 2 g 5% Pd/C-Katalysator (50% wasserfeucht) werden in einen 2L-Stahlautoklaven mit Begasungsrührer gegeben. Bei einer Temperatur von 25°C und einer Rührgeschwindigkeit von 800 U/min werden 10 bar Wasserstoff aufgepreßt. Verbrauchter Wasserstoff wird durch wiederholtes Aufpressen ersetzt. Nach 15 min läßt die Wasserstoffaufnahme nach. Der Katalysator wird bei Raumtemperatur über eine Drucknutsche abfiltriert. Man erhält 735,8 g einer Rohlösung, von der im Scheidetrichter 7,1 g Toluol abgetrennt werden. Die wäßrige Rohlösung wird in einem 1 L-Vierhalskolben mit pH-Meter auf 0°C abgekühlt. Der pH liegt bei 14. Innerhalb von 5 min werden 31,3 g (0,3 mol) Essigsäureanhydrid zugetropft, der pH fällt auf 6,0.).
Anschließend wird die Lösung langsam mit 49,3 g (0,5 mol) Salzsäure (37%ig) versetzt (pH 1,1). Dabei fällt ein weißer Niederschlag aus. Die Suspension wird noch eine halbe Stunde gerührt, dann wird der Niederschlag abfiltriert und zweimal mit 50 g Eiswasser gewaschen. Nach dem Trocknen erhält man 37,5 g (93,7 % d. Th.) 3-Acetoxy-2-methylbenzoesäure (Schmelzpunkt: 147°C)

## Patentansprüche

1. Verfahren zur Herstellung von 3-Hydroxy-2-methylbenzoesäure und 3-Acetoxy-2-methylbenzoesäure, dadurch gekennzeichnet, daß man 3-Chlor-2-methylphenol (3) 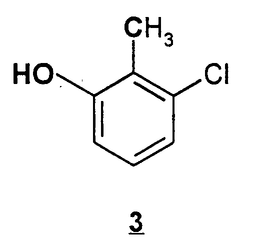 mit Benzylchlorid zu 2-Benzyloxy-6-chlortoluol (4) 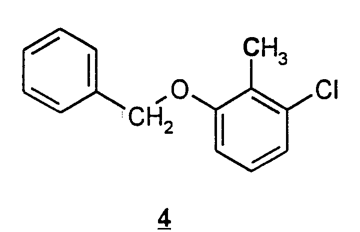 umsetzt, dieses mit Magnesium zu (3-Benzyloxy-2-methylphenyl)-magnesiumchlorid (5) 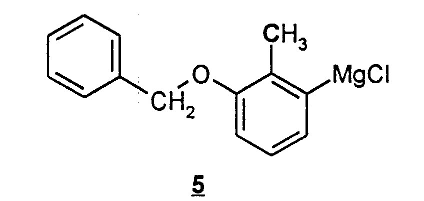 grignardisiert, dieses mit CO₂ zu 3-Benzyloxy-2-methylbenzoesäure (6) 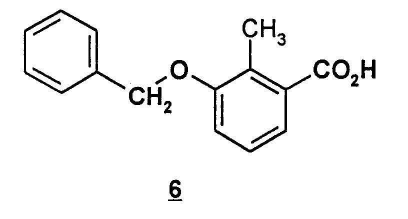 umsetzt, dieses oder seine Alkalimetallsalze im Gegenwart eines Hydrierungskatalysators zu 3-Hydroxy-2-methylbenzoesäure (2) hydriert und dieses gegebenenfalls zu 3-Acetoxy-2-methylbenzoesäure acetyliert.

2. Verfahren nach Anspruch 1, dadurch gekennzeichnet, daß Methylethylketon als Reaktionsmedium bei der Benzylierung von 3-Chlor-2-methylphenol (3) zu 2-Benzyloxy-6-chlortoluol (4) eingesetzt wird.

3. Verfahren nach Anspruch 1 oder 2, dadurch gekennzeichnet, daß pro Mol 3-Chlor-2-methylphenol (3) 1.0 bis 5.0 Mol, bevorzugt 1.1 bis 1.2 Mol Benzylchlorid, und 1.0 bis 5.0 Mol, bevorzugt 2.0 bis 2.5 Mol Kaliumcarbonat bei der Benzylierung von 3-Chlor-2-methylphenol (3) zu 2-Benzyloxy-6-chlortoluol (4) eingesetzt werden.

4. Verfahren nach Anspruch 1 bis 3, dadurch gekennzeichnet, daß die Umsetzung von 2-Benzyloxy-6-chlortoluol (4) mit Magnesium zu (3-Benzyloxy-2-methylphenyl)-magnesiumchlorid (5) in Tetrahydrofuran als Reaktionsmedium durchgeführt wird.

5. Verfahren nach Anspruch 1 bis 4, dadurch gekennzeichnet, daß der Hydrierungskatalysator Palladium auf Aktivkohle ist.

6. Verfahren nach dem Anspruch 1 bis 5, dadurch gekennzeichnet, daß die Hydrierung des Natrium-Salzes von 3-Benzyloxy-2-methylbenzoesäure (6) zu 3-Hydroxy-2-methylbenzoesäure (2) und Toluol in wäßrigem Medium durchgeführt wird.

7. Verfahren nach Anspruch 1 bis 6, dadurch gekennzeichnet ist, daß man die nach Anspruch 1-4 gewonnene 3-Benzyloxy-2-methylbenzoesäure (6) im basischen wäßrigen Medium im Gegenwart eines Hydrierungskatalysators zu 3-Hydroxy-2-methylbenzoesäure (2) und Toluol hydriert, den Katalysator durch Filtration abtrennt, die wäßrige Phase von der Toluol Phase abtrennt, mit Acetanhydrid umsetzt und das Reaktionsgemisch ansäuert.

8. 2-Benzyloxy-6-chlortoluol (4)

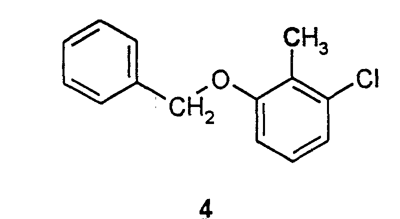

9. (3-Benzyloxy-2-methylphenyl)-magnesiumchlorid (5)

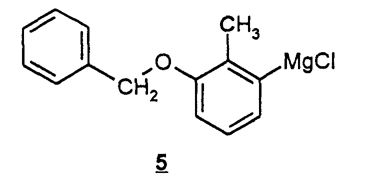

10. 3-Benzyloxy-2-methylbenzoesäure (6)

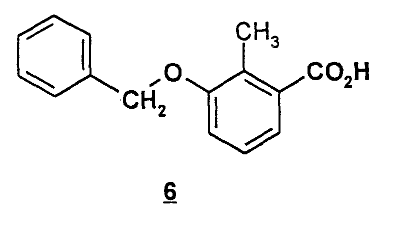

## Claims

1. A process for the preparation of 3-hydroxy-2-methylbenzoic acid and 3-acetoxy-2-methylbenzoic acid, which comprises reacting 3-chloro-2-methylphenol (3) 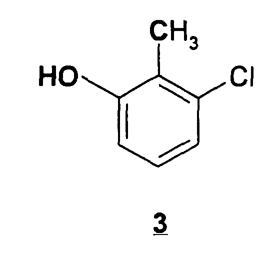 with benzyl chloride to give 2-benzyloxy-6-chlorotoluene (4) 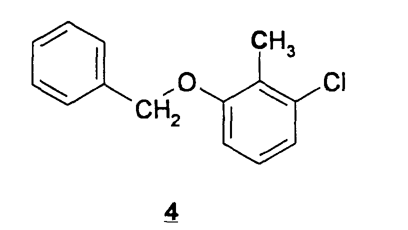 subjecting this to a Grignard reaction with magnesium to give (3-benzyloxy-2-methylphenyl)-magnesium chloride (5) 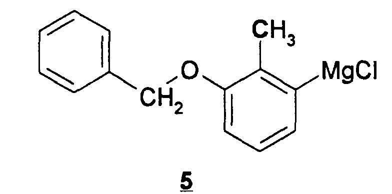 reacting this with CO₂ to give 3-benzyloxy-2-methylbenzoic acid (6) 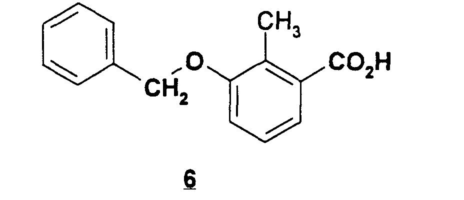 hydrogenating this or its alkali metal salts in the presence of a hydrogenation catalyst to give 3-hydroxy-2-methylbenzoic acid (2) and optionally acetylating this to give 3-acetoxy-2-methylbenzoic acid.

2. The process as claimed in claim 1, wherein methyl ethyl ketone is employed as a reaction medium in the benzylation of 3-chloro-2-methylphenol (3) to 2-benzyloxy-6-chlorotoluene (4).

3. The process as claimed in claim 1 or 2, wherein per mole of 3-chloro-2-methylphenol (3), 1.0 to 5.0 mol, preferably 1.1 to 1.2 mol, of benzyl chloride and 1.0 to 5.0 mol, preferably 2.0 to 2.5 mol, of potassium carbonate are employed in the benzylation of 3-chloro-2-methylphenol (3) to 2-benzyloxy-6-chlorotoluene (4).

4. The process as claimed in claims 1 to 3, wherein the reaction of 2-benzyloxy-6-chlorotoluene (4) with magnesium to give (3-benzyloxy-2-methylphenyl)-magnesium chloride (5) is carried out in tetrahydrofuran as reaction medium.

5. The process as claimed in claims 1 to 4, wherein the hydrogenation catalyst is palladium on active carbon.

6. The process as claimed in claims 1 to 5, wherein the hydrogenation of the sodium salt of 3-benzyloxy-2-methylbenzoic acid (6) to 3-hydroxy-2-methylbenzoic acid (2) and toluene is carried out in aqueous medium.

7. The process as claimed in claims 1 to 6, wherein the 3-benzyloxy-2-methylbenzoic acid (6) obtained as claimed in claims 1-4 is hydrogenated to 3-hydroxy-2-methylbenzoic acid (2) and toluene in the basic aqueous medium in the presence of a hydrogenation catalyst, the catalyst is separated off by filtration, the aqueous phase is separated off from the toluene phase and reacted with acetic anhydride, and the reaction mixture is acidified.

8. 2-Benzyloxy-6-chlorotoluene (4)

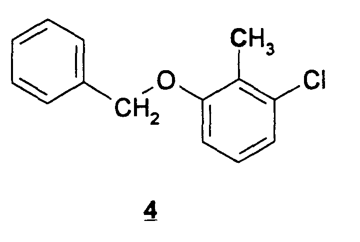

9. (3-Benzyloxy-2-methylphenyl)magnesium chloride (5)

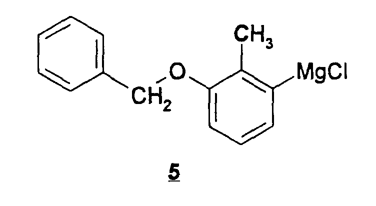

10. 3-Benzyloxy-2-methylbenzoic acid (6)

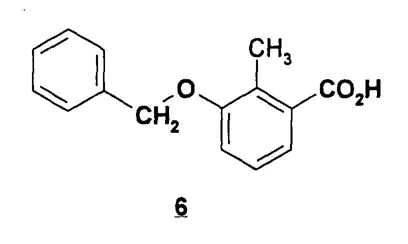

## Revendications

1. Procédé pour la préparation d'acide 3-hydroxy-2-méthylbenzoïque et d'acide 3-acétoxy-2-méthylbenzoïque, caractérisé en ce qu'on fait réagir le 3-chloro-2-méthylphénol (3) 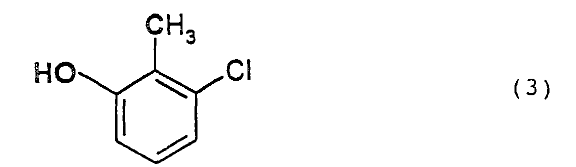 sur du chlorure de benzyle pour obtenir le 2-benzyl-oxy-6-chlorotoluène (4) 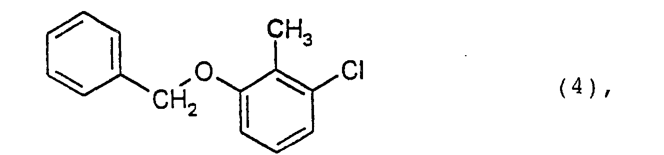 on grignardise celui-ci avec du magnésium pour obtenir le chlorure de (3-benzyloxy-2-méthylphényl)magnésium (5) 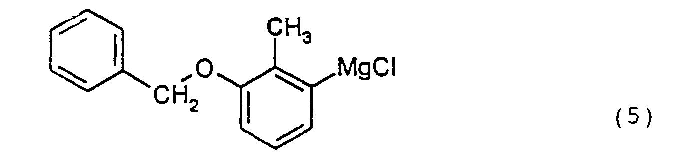 on fait réagir celui-ci sur CO₂ pour obtenir l'acide 3-benzyloxy-2-méthylbenzoïque (6) 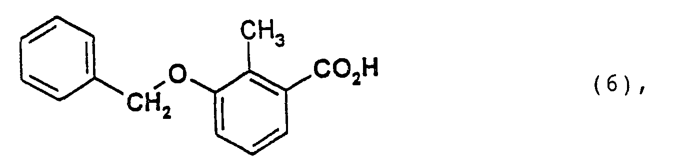 on hydrogène celui-ci ou ses sels de métaux alcalins en présence d'un catalyseur d'hydrogènation pour obtenir l'acide 3-hydroxy-2-méthylbenzoïque (2) et éventuellement on acétyle celui-ci pour obtenir l'acide 3-acétoxy-2-méthylbenzoïque.

2. Procédé selon la revendication 1, caractérisé en ce qu'on utilise la méthyléthylcétone comme milieu réactionnel dans la benzylation du 3-chloro-2-méthylphénol (3) en 2-benzyloxy-6-chloro-toluène (4).

3. Procédé selon la revendication 1 ou 2, caractérisé en ce qu'on utilise par mole de 3-chloro-2-méthylphénol (3) de 1,0 à 5,0 moles, de préférence de 1,1 à 1,2 mole, de chlorure de benzyle, et de 1,0 à 5,0 moles, de préférence de 2,0 à 2,5 moles, de carbonate de potassium dans la benzylation de 3-chloro-2-méthylphénol (3) en 2-benzyloxy-6-chloro-toluène (4).

4. Procédé selon la revendication 1 à 3, caractérisé en ce qu'on met en oeuvre la la réaction du 2-benzyloxy-6-chlorotoluène (4) sur la magnésium en chlorure de (3-benzyloxy-2-méthylphényl)magnésium (5) dans le tétrahydrofuranne comme milieu réactionnel.

5. Procédé selon la revendication 1 à 4, caractérisé en ce que le catalyseur d'hydrogénation est le palladium sur charbon actif.

6. Procédé selon la revendication 1 à 5, caractérisé en ce qu'on met en oeuvre l'hydrogénation du sel de sodium d'acide 3-benzyloxy-2-méthylbenzoïque (6) en acide 3-hydroxy-2-méthylbenzoïque (2) et toluène en milieu aqueux.

7. Procédé selon la revendication 1 à 6, caractérisé en ce qu'on met en oeuvre l'hydrogénation de l'acide 3-benzyloxy-2-méthylbenzoïque (6) obtenu selon la revendication 1 à 4, en acide 3-hydroxy-2-méthyl-benzoïque (2) et toluène en milieu aqueux basique en présence d'un catalyseur d'hydrogénation, on sépare le catalyseur par filtration, on sépare de la phase toluène la phase aqueuse, on fait réagir sur l'anhydride acétique et on acidifie le mélange réactionnel.

8. 2-Benzyloxy-6-chlorotoluène (4)

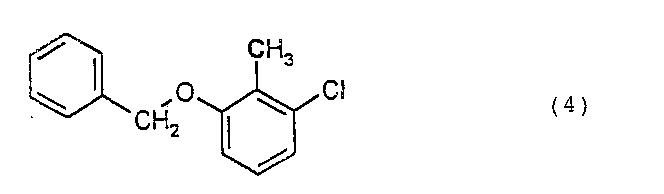

9. Chlorure de (3-benzyloxy-2-méthyl-phényl)-magnésium (5)

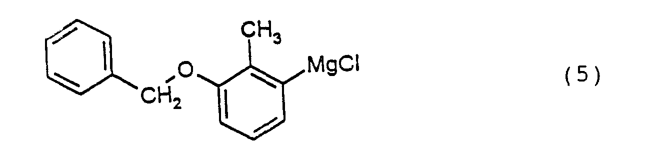

10. Acide 3-benzyloxy-2-méthylbenzoïque (6)
